# EUROPEAN PATENT SPECIFICATION

(11) **EP 3 656 412 B1**
(45) Date of publication and mention of the grant of the patent: **18.01.2023**
(21) Application number: 19210426.3
(22) Date of filing: 20.11.2019
(51) Int. Cl.: A61M 1/16, A61M 1/34

(54) **FLOW RATE CALIBRATION DEVICE OF FLUID REPLACEMENT PUMP IN HEMODIALYSIS SYSTEM**
FLUSSRATENKALIBRIERUNGSVORRICHTUNG FÜR EINE SUBSTITUTIONSPUMPE EINES HÄMODIALYSESYSTEMS
DISPOSITIF D'ÉTALONNAGE DE DÉBIT D'UNE POMPE DE REMPLACEMENT DE FLUIDE DANS UN SYSTÈME D'HÉMODIALYSE

(30) Priority: 22.11.2018 JP 2018219646
(43) Date of publication of application: 27.05.2020
(73) Proprietor: Shibuya Corporation, Kanazawa-shi, Ishikawa 920-8681 (JP)
(72) Inventor: TAKEMURA, Hideo, Kanazawa-shi, Ishikawa 920-8681 (JP)
(74) Representative: Mewburn Ellis LLP

(56) References cited:
- EP-A1- 0 321 754
- EP-B1- 0 321 754
- US-A- 5 200 090
- US-B2- 8 753 515

## Description

### BACKGROUND OF THE INVENTION

### Field of the Invention

The present invention relates to a fluid replacement pump for use in a hemodialysis system, herein referred to as a hemodialyzer, and more particularly to a flow rate calibration device that calibrates a flow rate of the fluid replacement pump.

### Description of the Related Art

Heretofore, a hemodialyzer comprising a fluid replacement pump has been known as a hemodialyzer (Japanese Patent No. 5858868, Japanese Patent No. 3180309).

A hemodialyzer described in Japanese Patent No. 5858868 comprises a dialysate chamber divided into a supply chamber and a recovery chamber by a diaphragm, a dialysate supply passage connecting the above supply chamber and a dialyzer, a dialysate recovery passage connecting the above dialyzer and the recovery chamber, a dialysate pump provided in the above dialysate recovery passage, a blood circuit connected to the above dialyzer, a fluid replacement passage connected to the above dialysate passage and the blood circuit, a fluid replacement pump provided in the above fluid replacement passage, and control means for controlling the above dialysate pump and the fluid replacement pump.

It is disclosed that a hemodialyzer described in Japanese Patent No. 3180309 detects a supply rate of a fluid replacement pump by use of a weighing container for exclusive use.

A flow rate of the above fluid replacement pump is directly associated with a fluid replacement rate in performing fluid replacement during a dialysis treatment, and hence it is desirable that the flow rate is accurate.

In Japanese Patent No. 3180309, a supply rate of the fluid replacement pump is directly detected using a weighing container for exclusive use, and hence the fluid replacement rate can be accurately grasped. However, there are disadvantages that the use of the weighing container incurs cost increase, and increases labor in cleaning.

The present invention provides a flow rate calibration device of a fluid replacement pump in a hemodialyzer in which a flow rate of the fluid replacement pump can be calibrated to perform fluid replacement at an accurate fluid replacement rate without especially incurring cost increase or increase of labor in cleaning.
EP 0321754 A1 discloses a method for monitoring haemodiafiltration in which an initial test is conducted on an ultrafiltrate pump on the one hand and on a balance on the other hand, using a monitoring device.
US 8753515 B2 discloses systems and methods for performing hemodialysis that include fluid handling systems that provide accurate control over the type and level of hemodialysis being performed.
US 5200090 discloses a system for weighing and monitoring flow from multiple fluid sources into a flow system.

### SUMMARY OF THE INVENTION

The invention is defined by the features of independent claim 1.

The invention of claim 1 provides a calibration device of a fluid replacement pump in a hemodialysis system comprising a dialysate chamber divided into a supply chamber and a recovery chamber by a diaphragm, a dialysate supply passage connecting the supply chamber and a dialyzer, a dialysate recovery passage connecting the dialyzer and the recovery chamber, a dialysate pump provided in the dialysate recovery passage, a blood circuit connected to the dialyzer, a fluid replacement passage connected to the dialysate supply passage and the blood circuit, a fluid replacement pump provided in the fluid replacement passage, and control means for controlling the dialysate pump and the fluid replacement pump, characterized in that the control means executes:
a first operation of operating the dialysate pump at a predetermined set flow rate to obtain total discharge time until dialysate supplied to fill the supply chamber runs out,
a second operation of operating the fluid replacement pump at a predetermined set flow rate only for predetermined time, to discharge a part of the dialysate supplied to fill the supply chamber,
a third operation of operating the dialysate pump at the same set flow rate as in the first operation, to obtain remaining discharge time until residual dialysate remaining in the supply chamber runs out after the end of the second operation, and
a fluid replacement pump calibrating operation of calculating a substantial flow rate of the fluid replacement pump from the total discharge time and the remaining discharge time, and adjusting setting of the fluid replacement pump to eliminate a difference in a case where there is the difference between the set flow rate and the substantial flow rate in the fluid replacement pump.

In the invention of claim 1, the control means first executes the first operation of operating the fluid replacement pump at the predetermined set flow rate to obtain the total discharge time until the dialysate supplied to fill the supply chamber runs out.

Next, the control means executes the second operation of operating the fluid replacement pump at the predetermined set flow rate only for the predetermined time, to discharge a part of the dialysate supplied to fill the supply chamber, and further continuously executes the third operation of operating the dialysate pump at the predetermined set flow rate again, to obtain the remaining discharge time until the residual dialysate remaining in the supply chamber runs out after the end of the second operation.

A maximum inner volume of the supply chamber is known, and hence a discharge amount of the dialysate discharged from the supply chamber by the fluid replacement pump can be calculated from the total discharge time and the remaining discharge time. Therefore, the substantial flow rate of the fluid replacement pump can be calculated from the discharge amount.

Consequently, in the calibrating operation of the fluid replacement pump, the control means can adjust the setting of the fluid replacement pump so that the difference is zero in the case where there is the difference between the set flow rate and the substantial flow rate in the fluid replacement pump.

Furthermore, in the present invention, a conventional configuration of the hemodialyzer can be used as it is, and hence inexpensive execution is possible.

### BRIEF DESCRIPTION OF THE DRAWINGS

FIG. 1 is a circuit diagram of a hemodialysis system (hemodialyzer) according to an embodiment of the present invention;
FIG. 2 is a circuit diagram explaining a first operation and a third operation of discharging dialysate in a supply chamber 21a to a recovery chamber 21b by a dialysate pump 27; and
FIG. 3 is a circuit diagram explaining a second operation of discharging the dialysate in the supply chamber 21a to a blood circuit 3 via a fluid replacement passage 6 by a fluid replacement pump 19.

### DETAILED DESCRIPTION OF THE PREFERRED EMBODIMENTS

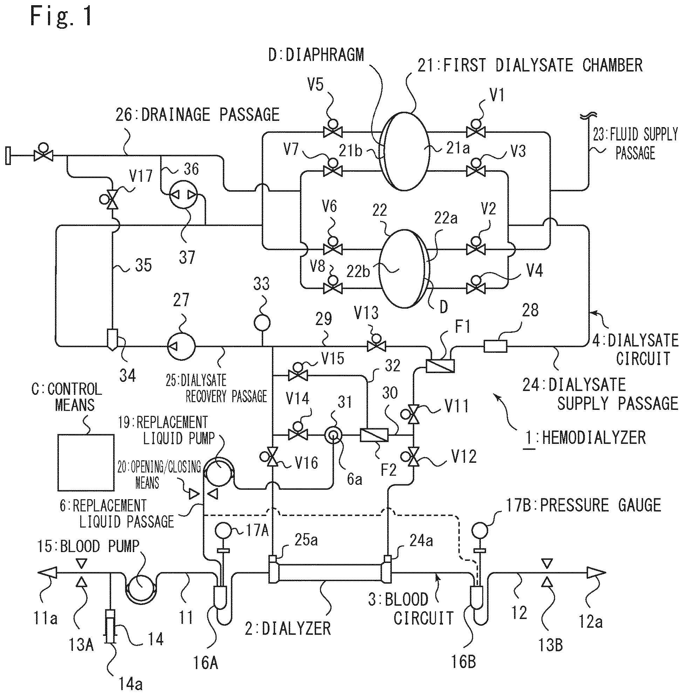

Hereinafter, description will be made as to an illustrated embodiment of the present invention. In FIG. 1, a hemodialyzer 1 to perform hemodialysis comprises a dialyzer 2 that performs the hemodialysis, a blood circuit 3 connected to the dialyzer 2, and a dialysate circuit 4 connected to the dialyzer 2.

The hemodialyzer 1 of the embodiment of the present invention is capable of performing an on-line HDF or HF to supply fresh dialysate to a patient via the blood circuit 3, and the hemodialyzer comprises a fluid replacement passage 6 branched from the blood circuit 3 and connected to the dialysate circuit 4.

The blood circuit 3 comprises an artery side passage 11 connected to patient's artery and connected to one end of the dialyzer 2, and a vein side passage 12 connected to a vein and connected to the other end of the dialyzer 2. The fluid replacement passage 6 is connected to branch from the artery side passage 11 in the illustrated embodiment. Note that as shown by a dotted line of FIG. 1, the fluid replacement passage 6 may be connected to branch from the vein side passage 12.

In the artery side passage 11, provided are a puncture needle 11a to puncture the patient, clamp means 13A for opening and closing the artery side passage 11, a syringe 14 containing an anticoagulant, a blood pump 15 comprising a tube pump (a peristaltic pump) that sends blood, and a drip chamber 16A that removes air from the blood, and in the drip chamber 16A, a pressure gauge 17A is provided.

A plunger 14a of the syringe 14 is provided with unshown pressing means for pressing this plunger. Thus, the pressing means presses the plunger 14a, so that the anticoagulant, such as heparin, can be supplied to the artery side passage 11.

In the vein side passage 12, provided are a drip chamber 16B that removes air from the blood, clamp means 13B for opening and closing the vein side passage 12, and a puncture needle 12a to puncture the patient, and in the drip chamber 16B, a pressure gauge 17B is provided.

As described above, the fluid replacement passage 6 is connected to the drip chamber 16A in the artery side passage 11, and this fluid replacement passage 6 is replaced, together with the dialyzer 2 and the blood circuit 3, every time the dialysis treatment is performed.

Furthermore, at an upstream end of the fluid replacement passage 6, provided is a coupler 6a to be connected to a fluid replacement port 31 that will be described later, and further in the fluid replacement passage 6, provided are a fluid replacement pump 19 comprising a tube pump in the same manner as in the blood pump 15, and opening/closing means 20, such as a pair of forceps, a clamp or Robert clamp, for opening and closing the fluid replacement passage 6.

Note that as described above, the fluid replacement passage 6 can be connected to the drip chamber 16B in the vein side passage 12 by judgment of a doctor in charge. Furthermore, the opening/closing means 20 is not limited to a shown position when provided, and may be provided at any position in the fluid replacement passage 6.

The dialysate circuit 4 comprises first and second dialysate chambers 21, 22 of the same shape that supply and discharge the dialysate, a fluid supply passage 23 that supplies the fresh dialysate to the first and second dialysate chambers 21, 22, a dialysate supply passage 24 that supplies fresh dialysate from the first and second dialysate chambers 21, 22 to the dialyzer 2, a dialysate recovery passage 25 that recovers the used dialysate passed through the dialyzer 2, in the first and second dialysate chambers 21, 22, and a drainage passage 26 that discharges the used dialysate from the first and second dialysate chambers 21, 22 to an unshown drainage tank.

In the present embodiment, a pump or an on-off valve that forms the dialysate circuit 4 as described below forms dialysate distribution means for distributing the dialysate through the dialysate circuit 4, and this means is controlled by control means C.

An interior of each of the first dialysate chamber 21 and the second dialysate chamber 22 is divided into two chambers by one diaphragm D, and supply chambers 21a, 22a containing the fresh dialysate and recovery chambers 21b, 22b containing the used dialysate are defined and formed.

In the fluid supply passage 23, an unshown fluid supply pump that supplies the fresh dialysate is provided, and the pump has a downstream portion branched in two directions and connected to the supply chambers 21a, 22a of the first and second dialysate chambers 21, 22. Furthermore, the branched portions are provided with fluid supply valves V1, V2, respectively.

The dialysate supply passage 24 has an upstream portion branched in two directions and connected to the supply chambers 21a, 22a of the first and second dialysate chambers 21, 22, and the passage has a downstream end provided with a coupler 24a to be connected to the dialyzer 2. Furthermore, the branched portions are provided with supply valves V3, V4, respectively, and a flow switch 28 that detects presence or absence of distribution of the dialysate is provided on a downstream side of the branched portions.

The dialysate recovery passage 25 has an upstream end provided with a coupler 25a to be connected to the dialyzer 2, and the passage has a downstream portion branched in two directions and connected to the recovery chambers 21b, 22b of the first and second dialysate chambers 21, 22.

Furthermore, a dialysate pump 27 that supplies the dialysate is provided on an upstream side of the branched portions, in the dialysate recovery passage 25, and the branched portions are provided with recovery valves V5, V6, respectively.

The drainage passage 26 has an upstream portion branched in two directions and connected to the recovery chambers 21b, 22b of the first and second dialysate chambers 21, 22, and the passage has a downstream portion connected to an unshown waste liquid tank. Furthermore, portions branched as described above are provided with drain valves V7, V8, respectively.

Furthermore, in the dialysate supply passage 24, a first dialysate filter F1 that removes a harmful component of the dialysate and first and second on-off valves V11, V12 arranged away from each other at a predetermined interval are provided on a downstream side of the flow switch 28.

The first dialysate filter F1 is divided into an upstream chamber and a downstream chamber by a semipermeable membrane, to remove the harmful component from the dialysate that is passed from the upstream chamber through the semipermeable membrane to the downstream chamber.

The upstream chamber of the first dialysate filter F1 is connected to a first bypass passage 29 via which the dialysate supply passage 24 is in communication with the dialysate recovery passage 25, and the first bypass passage 29 is provided with a third on-off valve V13.

Furthermore, a second bypass passage 30 via which the dialysate supply passage 24 is in communication with the dialysate recovery passage 25 is connected between the first on-off valve V11 and the second on-off valve V12, and the second bypass passage 30 is provided with a second dialysate filter F2, the fluid replacement port 31 connected to the fluid replacement passage 6, and a fourth on-off valve V14 in order from the upstream side.

The second dialysate filter F2 is also divided into an upstream chamber and a downstream chamber by a semipermeable membrane in the same manner as in the first dialysate filter F1, and the upstream chamber is connected, via the second bypass passage 30, to a third bypass passage 32 via which the dialysate supply passage 24 is in communication with the dialysate recovery passage 25. The third bypass passage 32 is provided with a fifth on-off valve V15.

The fluid replacement port 31 is connected to the coupler 6a of the fluid replacement passage 6 during the dialysis treatment, so that the dialysate can be supplied to the fluid replacement passage 6. Furthermore, during preparation for the dialysis treatment, the fluid replacement passage 6 is not connected, and the dialysate is distributed to the first bypass passage 29 as it is.

On the other hand, the dialysate recovery passage 25 is provided with a sixth on-off valve V16, a pressure sensor 33 that measures a pressure, the dialysate pump 27 that supplies the dialysate, and an air removal tank 34 that removes air from the dialysate in order from a dialyzer 2 side.

The first bypass passage 29 has a downstream end connected to a position adjacent to an upstream side of the pressure sensor 33, and the second bypass passage 30 has a downstream end connected to a position adjacent to a downstream side of the sixth on-off valve V16. The third bypass passage 32 has a downstream end connected between the first bypass passage 29 and the second bypass passage 30.

The air removal tank 34 is provided with a fourth bypass passage 35 via which the dialysate recovery passage 25 is in communication with the drainage passage 26, and the fourth bypass passage 35 is provided with a seventh on-off valve V17.

Furthermore, on a downstream side of the air removal tank 34, a fifth bypass passage 36 is provided via which the dialysate recovery passage 25 is in communication with the drainage passage 26, and the fifth bypass passage 36 is provided with a water removal pump 37 to remove water during the dialysis treatment.

In the above described configuration, in a state immediately before the control means C is commanded to start the dialysis treatment, the puncture needles 11a, 12a of the blood circuit 3 are connected to the patient. The control means C also operates the fluid supply pump of the fluid supply passage 23 and the dialysate pump 27 of the dialysate recovery passage 25, and controls opening/closing of various valves described above. Furthermore, the opening/closing means 20 is opened by a manual operation to open the fluid replacement passage 6. At this time, the dialysate pump 27 is operated at a predetermined flow rate required for the dialysis treatment, for example, a set flow rate of 100 ml/min by the control means C, while the water removal pump 37 and the fluid replacement pump 19 are stopped.

In a state where the fluid supply pump of the fluid supply passage 23 and the dialysate pump 27 are operated, the dialysate is supplied up to a maximum volume to fill the supply chamber 21a of one dialysate chamber 21. In this case, the diaphragm D is moved to a left end of FIG. 1, and hence a volume of the recovery chamber 21b of the dialysate chamber 21 is zero. At this time, a volume of the supply chamber 22a of the other dialysate chamber 22 is zero, and a volume of the recovery chamber 22b of the dialysate chamber 22 is maximum (a state of FIG. 1).

At this moment, the distribution of the dialysate through the dialysate circuit 4 is stopped, and the flow switch 28 is therefore in an off-state. When the flow switch 28 turns off, the control means C opens the supply valve V3 and the recovery valve V5 on a first dialysate chamber 21 side, and closes the fluid supply valve V1 and the drain valve V7. Then, the used dialysate distributed through the dialyzer 2 is sent to the recovery chamber 21b by the dialysate pump 27, and the diaphragm D is accordingly deformed to decrease the volume of the supply chamber 21a. Consequently, the fresh dialysate contained in the supply chamber 21a flows through the dialysate supply passage 24 and is supplied to the dialyzer 2.

When the dialysate flows through the dialysate circuit 4 in this manner, the flow switch 28 is in an on-state.

As described above, the control means C opens the supply valve V3 and the recovery valve V5 on the first dialysate chamber 21 side, and closes the fluid supply valve V1 and the drain valve V7. The control means simultaneously opens the fluid supply valve V2 and the drain valve V8, and closes the supply valve V4 and the recovery valve V6 on the other side, i.e., a second dialysate chamber 22 side. Consequently, the fresh dialysate flows from the fluid supply passage 23 into the supply chamber 22a. The diaphragm D is accordingly deformed to decrease the volume of the recovery chamber 22b, and the used dialysate contained in the recovery chamber 22b is discharged to outside via the drainage passage 26.

The recovery chamber 21b of the first dialysate chamber 21 is filled with the used dialysate, and the volume of the supply chamber 21a is zero. Then, an amount of the dialysate flowing through the dialysate circuit 4 is zero, and hence the flow switch 28 is in the off-state.

An amount of the dialysate to be supplied by the unshown fluid supply pump provided in the fluid supply passage 23 is set to be larger than an amount of the dialysate to be supplied by the dialysate pump 27 of the dialysate recovery passage 25. Consequently, the recovery chamber 21b of the first dialysate chamber 21 is filled with the used dialysate, and the supply chamber 22a of the second dialysate chamber 22 on the other side is filled with the fresh dialysate before the volume of the supply chamber 21a turns to zero. Therefore, the volume of the recovery chamber 22b is zero.

Consequently, in the off-state of the flow switch 28, the control means C switches the fluid supply valves V1, V2, the supply valves V3, V4, the recovery valves V5, V6, and the drain valves V7, V8, to supply the fresh dialysate from the supply chamber 22a of the second dialysate chamber 22 to the dialyzer 2 and to recover the used dialysate into the recovery chamber 22b. Furthermore, the fresh dialysate is supplied from the supply passage 23 to the supply chamber 21a of the first dialysate chamber 21, and the used dialysate from the recovery chamber 21b is discharged from the drainage passage 26 to the outside.

Thus, every time the flow switch 28 turns off, the control means C alternately opens and closes the fluid supply valves V1, V2, the supply valves V3, V4, the recovery valves V5, V6, and the drain valves V7, V8, to continuously supply the fresh dialysate from the first and second dialysate chambers 21, 22 to the dialyzer 2 via the dialysate supply passage 24 and to recover the used dialysate passed through the dialyzer 2 into the first and second dialysate chambers 21, 22 via the dialysate recovery passage 25. Consequently, the distribution of the dialysate is maintained.

### (Start of Dialysis Treatment)

When the control means C is commanded to start the dialysis treatment from a prepared state for the dialysis treatment described above, the control means C waits for either one of the supply chamber 21a or 22a to be filled with the dialysate, and then starts the dialysis treatment.

In this dialysis treatment, the control means C starts the operation of the water removal pump 37, and discharges the used dialysate outward from the dialysate circuit 4 to the drainage passage 26 at a predetermined flow rate. An amount of the water to be discharged by the water removal pump 37 is an amount of water containing wastes to be discharged from the blood to the outside via the dialyzer 2, i.e., an amount of the water to be removed.

### (Blockage Detecting Operation of Fluid Replacement Passage 6)

When the control means C is given a fluid replacement command to execute the fluid replacement, for example, at a flow rate of 50 ml/min for 30 minutes during the above described dialysis treatment, the control means C first detects presence or absence of blockage in the fluid replacement passage 6. For example, it is detected whether the opening/closing means 20 such as the pair of forceps, clamp or Robert clamp to close the fluid replacement passage 6 is forgotten to be removed or operated and whether the fluid replacement passage 6 remains closed.

### (First Operation: Measuring of Total Discharge Time)

At a suitable time, the control means C measures and stores time from a moment when the flow switch 28 turns on to a moment when the flow switch 28 turns off, i.e., total discharge time until a total amount of the dialysate supplied to fill the supply chamber 21a or 22a is discharged to a recovery chamber 21b or 22b side.

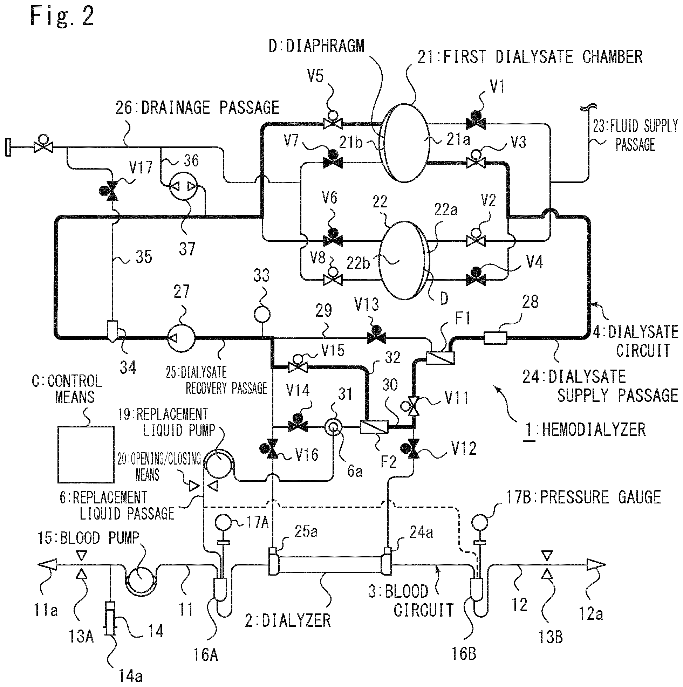

At this time, for example, when the supply chamber 21a is full and the flow switch 28 is off, the control means C opens the supply valve V3, the recovery valve V5 and the on-off valve V15, and closes the on-off valves V12, V14 and V16 as shown in FIG. 2. Then, the total amount of the dialysate in the supply chamber 21a is supplied to the recovery chamber 21b via the second bypass passage 30 and the third bypass passage 32 without being passed through the dialyzer 2. Furthermore, in this case, the control means C stops the operation of the water removal pump 37.

Consequently, when the dialysate in the supply chamber 21a starts to be supplied at a supply rate of 100 ml/min by the dialysate pump 27, the flow switch 28 turns on. Therefore, the control means C measures the total discharge time from this moment to a moment when the dialysate in the supply chamber 21a runs out and the flow switch 28 turns off. For example, in a case where the measured total discharge time is one minute, this is stored as the total discharge time.

### (Calibrating Operation of Dialysate Pump 27)

Incidentally, in a case where a maximum volume of the supply chamber 21a is 100 ml and the total discharge time is one minute, a substantial flow rate of the dialysate pump 27 can be calculated as 100 ml/min. This numeric value matches the above described flow rate of the dialysate pump 27 that is set by the control means C, and hence it can be determined that the dialysate pump 27 is operated at a correct set flow rate. Note that two supply chambers 21a, 22a are manufactured to substantially have the same predetermined volume, and hence the discharge time in a case where the dialysate is supplied from either one of the supply chambers may be measured. However, a volume of the dialysate chamber 21, 22 may be different from a designed volume due to a manufacturing error or the like. Consequently, each volume may be accurately measured and input into the control means C, and either one supply chamber for use may be managed by the control means C.

In this case, if the total discharge time is 61 seconds, it is calculated that the substantial flow rate of the dialysate pump 27 is 98.4 ml/min. Therefore, it can be determined that there is an error from the set flow rate. To solve this problem, the control means C adjusts a rotation number, a voltage or the like of the dialysate pump 27 to perform calibration so that an actual flow rate of the dialysate reaches a set flow rate of 100 ml/min.

Conversely, if the total discharge time is 59 seconds, the substantial flow rate of the dialysate pump 27 is 101.7 ml/min. Therefore, also in this case, the control means C performs the calibration so that the actual flow rate of the dialysate matches the set flow rate.

Thus, in the present embodiment, the flow rate of the dialysate pump 27 is calibrated based on the measured total discharge time, and hence calibrated discharge time of one minute is used as the total discharge time as described below.

Note that in the above embodiment, immediately after the control means C is given the fluid replacement command, the control means executes a first operation and a calibrating operation of the dialysate pump 27. In another hemodialyzer, however, the first operation and the calibrating operation of the dialysate pump 27 may be executed during the dialysis treatment.

That is, there may be a hemodialyzer that executes the first operation and the calibrating operation of the dialysate pump 27 every time the total amount of the dialysate is supplied from the supply chamber 21a or 22a to the recovery chamber 21b or 22b during the dialysis treatment (feedback control of the dialysate pump 27).

In this case, the above described execution of the first operation and the calibrating operation of the dialysate pump 27 immediately after the fluid replacement command is given may be omitted. The first operation and the calibrating operation of the dialysate pump 27 that are executed finally during the dialysis treatment can be used, to immediately execute a discharge operation by the fluid replacement pump 19 as will be described below.

### (Discharge by Fluid Replacement Pump 19)

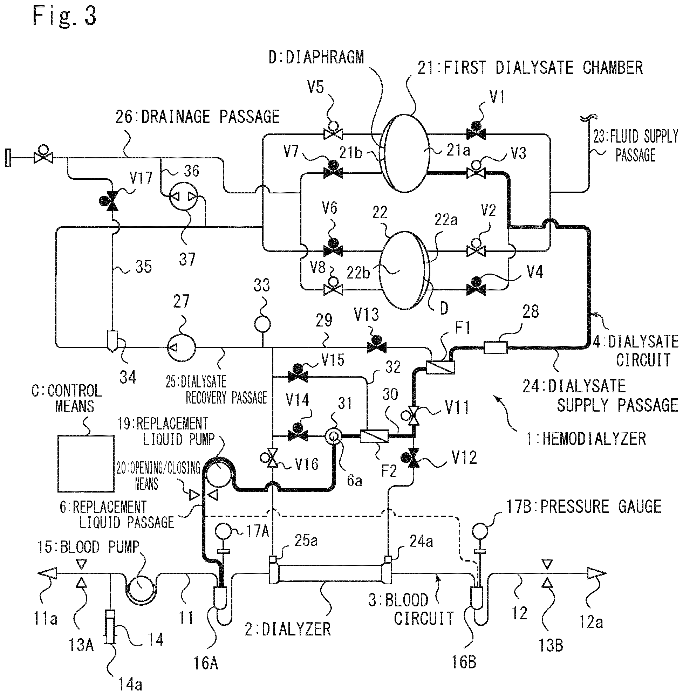

After end of the above first operation, the control means C continuously waits for either one supply chamber 21a or 22a to be filled with the dialysate. Then, for example, the supply chamber 21a is filled with the dialysate, and the dialysate starts to be supplied from the supply chamber 21a to the dialysate supply passage 24. As shown in FIG. 3, the control means simultaneously closes the previously open on-off valves V12, V15, and starts the operation of the fluid replacement pump 19 at a flow rate of 50 ml/min that is a set flow rate based on fluid replacement command conditions.

When the fluid replacement pump 19 is operated, the dialysate in the supply chamber 21a is supplied from the dialysate supply passage 24 via the fluid replacement passage 6 to the blood circuit 3. Consequently, flow is generated in the flow switch 28. The flow switch 28 is turned on by the dialysate flowing at a flow rate that is more than or equal to a minimum operation flow rate. Therefore, if the minimum operation flow rate of the flow switch 28 is 50 ml/min or less as described above, the flow switch 28 is turned on by the flow at the above flow rate of 50 ml/min based on the fluid replacement command conditions. In this case, it can be determined whether or not the fluid replacement passage 6 is blocked, by determining whether or not the flow switch 28 turns on.

However, in a case where the minimum operation flow rate of the flow switch 28 is 50 ml/min or more, the fluid replacement pump 19 is operated so that the dialysate flows through the flow switch 28, but the flow switch 28 does not turn on. Alternatively, in a case where the minimum operation flow rate of the flow switch 28 is 50 ml/min or less but a command flow rate in accordance with the fluid replacement command is set to be less than the minimum operation flow rate, the flow switch 28 does not turn on.

In consideration of such circumstances, in the present embodiment, the presence or absence of the blockage in the fluid replacement passage 6 can be securely detected as described below.

### (Second Operation)

That is, when given the command of the fluid replacement as described above, the control means C first operates the fluid replacement pump 19 at a predetermined set flow rate only for predetermined time in a state where the supply chamber 21a is filled with the dialysate. Consequently, the control means executes a second operation of discharging a part of the dialysate supplied to fill the supply chamber 21a to the blood circuit 3 via the fluid replacement passage 6.

More specifically, for example, the fluid replacement pump 19 is operated at a set flow rate of 50 ml/min for one minute, whereby the dialysate is discharged as much as an assumed amount of 50 ml from the supply chamber 21a. Consequently, an assumed amount of the residual dialysate remaining in the supply chamber 21a is a half, i.e., 50 ml.

At this time, when the inner volume of the supply chamber 21a actually decreases as much as 50 ml, the inner volume of the recovery chamber 21b increases as much as 50 ml. The increase in the inner volume of the recovery chamber 21b leads to a negative pressure in the dialyzer 2, and hence 50 ml of the water containing the wastes is filtered from the blood.

Note that the above term "assumed" is used because, if the fluid replacement passage 6 is completely blocked by the opening/closing means 20, an actual amount of the dialysate to be discharged is zero and the dialysate is not necessarily securely discharged.

Furthermore, it is preferable that the amount of the dialysate to be discharged from the supply chamber 21a by the fluid replacement pump 19 is about a half of the amount of the dialysate supplied to fill the supply chamber. This is because a small amount of the dialysate to be discharged from the supply chamber 21a by the fluid replacement pump 19 leads to a small difference between the remaining discharge time and the total discharge time, and due to a large amount of the dialysate to be discharged, a set flow rate of the fluid replacement pump is generally set to be smaller than a set flow rate of the dialysate pump. Consequently, time required for detection of blockage in the fluid replacement passage lengthens.

### (Third Operation: Measuring of Remaining Discharge Time)

With elapse of one minute described above, that is, after end of the second operation, the control means C again switches to a circuit shown in FIG. 2, to perform a third operation of discharging the residual dialysate remaining the supply chamber 21a to the recovery chamber 21b by the dialysate pump 27 until the residual dialysate runs out, and measuring the remaining discharge time.

That is, with the elapse of one minute described above, the control means C closes the on-off valve V16 and opens the on-off valve V15 to send the dialysate remaining in the supply chamber 21a to the recovery chamber 21b side. In this case, the dialysate remaining in the supply chamber 21a is supplied at a supply rate of 100 ml/min by the dialysate pump 27, and hence the flow switch 28 is securely in the on-state. Then, the control means C measures the remaining discharge time from this moment to a moment when the amount of the dialysate in the supply chamber 21a is zero and the flow switch 28 turns off.

At this time, in a case where it is detected that the remaining discharge time is one minute, this remaining discharge time matches the total discharge time in the first operation. Therefore, in this case, it is supposed that 50 ml of the dialysate is discharged from the supply chamber 21a by the second operation, but in actual, the dialysate is not discharged at all.

On the other hand, in a case where it is detected that the remaining discharge time is 30 seconds, the set flow rate of the dialysate pump 27 is 100 ml/min, and hence it can be calculated that a substantial amount of the residual dialysate remaining in the supply chamber 21a is 50 ml. If the fluid replacement pump 19 is operated for one minute and the fluid replacement passage 6 is not blocked, an assumed amount of the dialysate remaining in the supply chamber 21a is then supposed to be 50 ml. Therefore, in this case, it can be determined that the fluid replacement passage 6 is not blocked.

### (Fourth Operation: Determining Operation of Presence or Absence of Blockage in Fluid Replacement Passage)

As described above, the control means C compares the total discharge time measured in the first operation with the remaining discharge time in the third operation, and determines that the fluid replacement passage 6 is blocked when the difference between both the times is in the allowable error range.

In this fourth operation in which the control means C determines that the fluid replacement passage 6 is blocked, the control means gives a warning by display or sound to that effect, and maintains the hemodialyzer 1 in a state immediately before the above described start of the dialysis treatment is commanded.

There is assumed a case where the fluid replacement passage 6 is incompletely opened. For example, when it is detected that the remaining discharge time is 45 seconds, the passage is in an incomplete open state between complete blockage and compete open. A degree of incomplete open that is determined as "open" or "blockage" is set in consideration of various circumstances. If the remaining discharge time that is detected as 45 seconds is included in "open", the remaining discharge time detected as 45 seconds is included in the allowable error range.

That is, the allowable error range includes an error from an actual flow rate in a case where the dialysate pump 27 is operated at the set flow rate without being calibrated, and an error from an actual flow rate in a case where the fluid replacement pump 19 is operated at the set flow rate. Furthermore, the range also includes an error of the fluid replacement passage 6 that is not completely opened but can be determined to be almost completely opened.

Additionally, in a case of determination that the fluid replacement passage 6 is blocked, the control means C is again given the fluid replacement command by a manual operation of opening the passage with the opening/closing means 20, and repeats the above described operation again to determine the presence or absence of the blockage in the fluid replacement passage 6.

### (Calibrating Operation of Fluid Replacement Pump 19)

In a case of determination that the fluid replacement passage 6 is not blocked, the control means C performs the calibrating operation of the flow rate of the fluid replacement pump 19 by use of the remaining discharge time when there is not any blockage.

That is, in the calibrating operation of the fluid replacement pump 19, the control means calculates the substantial flow rate of the fluid replacement pump 19 from the set flow rate of the dialysate pump 27 and the remaining discharge time in the third operation. In a case where there is a difference between the set flow rate and the substantial flow rate in the fluid replacement pump 19, the control means calibrates the set flow rate of the fluid replacement pump so that the difference is zero.

More specifically, for the dialysate pump 27 having a set flow rate of 100 ml/min and a remaining discharge time of 30 seconds in the third operation, a substantial amount of the residual dialysate remaining in the supply chamber 21a can be calculated as 50 ml. Therefore, an amount of the dialysate to be discharged by the fluid replacement pump 19 in the second operation can be calculated as 50 ml. In this case, the fluid replacement pump 19 is operated at a set flow rate of 50 ml/min for one minute in the second operation, and hence the dialysate pump 27 is accurately operated at the set flow rate of 50 ml/min, thereby obviating the calibration.

On the other hand, if it is detected that the remaining discharge time is 31 seconds in the third operation in which the dialysate pump 27 has a set flow rate of 100 ml/min, the substantial amount of the residual dialysate remaining in the supply chamber 21a can be calculated as 51.7 ml. Therefore, it can be calculated that the amount of the dialysate to be discharged by the fluid replacement pump 19 in the second operation is 48.3 ml and the substantial flow rate of the fluid replacement pump 19 is 48.3 ml/min.

In this case, since there is a difference between the set flow rate of 50 ml/min and the substantial flow rate of 48.3 ml/min in the fluid replacement pump 19, the control means C calibrates the rotation number, the voltage or the like of the fluid replacement pump 19 so that the substantial flow rate matches the set flow rate.

In the second operation in which it is detected that the discharge time of the dialysate pump 27 is shorter than 30 seconds, the control means C also calibrates the rotation number, the voltage or the like of the fluid replacement pump 19 so that the substantial flow rate matches the set flow rate.

When the calibrating operation of the fluid replacement pump 19 is completed as described above, the control means C actually executes the above described fluid replacement command, that is, the command to execute the fluid replacement at the flow rate of 50 ml/min for 30 minutes.

In this case, the hemodialyzer 1 is returned to the above described execution of the dialysis treatment, and the operation of the calibrated fluid replacement pump 19 is continued to execute the fluid replacement for 30 minutes.

Then, at the end of the fluid replacement for the predetermined time, the operation of the fluid replacement pump 19 is stopped, thereby returning to the usual dialysis treatment.

As described above, in the present embodiment, since the fluid replacement pump 19 can be calibrated using the flow switch 28 for use in switching the dialysate chambers 21, 22, inexpensive execution is possible.

Note that in the above embodiment, for example, in the case where the inner volume of the supply chamber 21a is maximized, the inner volume of the supply chamber 21a turns to zero, or the dialysate in the supply chamber 21a runs out, a certain degree of error in the amount of the dialysate is included. As described above, the flow switch 28 turns on and off based on whether or not the dialysate flows through the switch at the flow rate that is more than or equal to the minimum operation flow rate, and the switch does not turn on and off based on whether or not the flow rate is completely zero.

Furthermore, in the above embodiment, the flow rate of the dialysate pump 27 is calibrated based on the measured total discharge time, but the flow rate of the dialysate pump 27 does not necessarily have to be calibrated. That is, in the above described embodiment, the total discharge time of 61 seconds or 59 seconds in the dialysate pump 27 prior to the calibration is adopted as the total discharge time as it is. The substantial flow rate of the dialysate pump 27 can be calculated as 98.4 ml/min or 101.7 ml/min from the total discharge time. Consequently, during the calibrating operation of the fluid replacement pump 19, the flow rate of the fluid replacement pump 19 can be accurately calibrated using the substantial flow rate as the set flow rate of the dialysate pump 27.

Furthermore, in the above embodiment, the control means is given the fluid replacement command and accordingly calibrates the flow rate of the fluid replacement pump 19. A plurality of calibrating operations of the flow rate of the fluid replacement pump 19 can be performed at suitable times. The fluid replacement passage 6 is replaced every time the dialysis treatment is performed. However, it is preferable to perform a plurality of replacements in the middle of the fluid replacement, because flexibility or the like of a tube that forms the fluid replacement passage 6 changes every moment.

Additionally, in the above embodiment, the flow switch 28 is used in the measurement of the time, but the present invention is not limited to the use of the flow switch 28, and any measuring means may be used as long as the time can be measured.

Furthermore, in the above embodiment, the dialysate chamber 21 or 22 is divided into two chambers of the supply chamber 21a or 22a and the recovery chamber 21b or 22b by the diaphragm D, but the present invention is not limited to this example. The present invention can be applied to a hemodialyzer shown in FIG. 9 of Japanese Patent No. 5858868 in which an interior of a dialysate chamber is divided into three chambers by two diaphragms D and a variable volume chamber is formed between a supply chamber and a recovery chamber.

### Reference Signs List

- 1: hemodialyzer
- 2: dialyzer
- 3: blood circuit
- 4: dialysate circuit
- 6: fluid replacement passage
- 19: fluid replacement pump
- 21 and 22: dialysate chamber
- 21a and 22a: supply chamber
- 21b and 22b: recovery chamber
- 24: dialysate supply passage
- 25: dialysate recovery passage
- 27: dialysate pump
- 28: flow switch
- C: control means
- D: diaphragm

## Claims

1. A calibration device of a fluid replacement pump in a hemodialysis system (1) comprising a dialysate chamber (21, 22) divided into a supply chamber (21a, 22a) and a recovery chamber (21b, 22b) by a diaphragm (D), a dialysate supply passage (24) connecting the supply chamber (21a, 22a) and a dialyzer (2), a dialysate recovery passage (25) connecting the dialyzer (2) and the recovery chamber (21b, 22b), a dialysate pump (27) provided in the dialysate recovery passage (25), a blood circuit (3) connected to the dialyzer (2), a fluid replacement passage (6) connected to the dialysate supply passage (24) and the blood circuit (3), the fluid replacement pump (19) provided in the fluid replacement passage (6), and control means (C) for controlling the dialysate pump (27) and the fluid replacement pump (19), **characterized in that** the control means (C) executes:
a first operation of operating the dialysate pump (27) at a predetermined set flow rate to obtain total discharge time until dialysate supplied to fill the supply chamber runs out,
a second operation of operating the fluid replacement pump (19) at a predetermined set flow rate only for predetermined time, to discharge a part of the dialysate supplied to fill the supply chamber (21a, 22a),
a third operation of operating the dialysate pump (27) at the same set flow rate as in the first operation, to obtain remaining discharge time until residual dialysate remaining in the supply chamber (21a, 22a) runs out after the end of the second operation, and
a fluid replacement pump calibrating operation of calculating a substantial flow rate of the fluid replacement pump (19) from the total discharge time and the remaining discharge time, and adjusting setting of the fluid replacement pump (19) to eliminate a difference in a case where there is the difference between the set flow rate and the substantial flow rate in the fluid replacement pump (19).

## Patentansprüche

1. Kalibrationsvorrichtung einer Fluidersatzpumpe in einem Hämodialysesystem (1), umfassend eine Dialysatkammer (21, 22), die durch eine Membran (D) in eine Zufuhrkammer (21a, 22a) und eine Rückgewinnungskammer (21b, 22b) unterteilt ist, einen Dialysatzufuhrkanal (24), der die Zufuhrkammer (21a, 22a) und ein Dialysegerät (2) verbindet, einen Dialysatrückgewinnungskanal (25), der das Dialysegerät (2) und die Rückgewinnungskammer (21b, 22b) verbindet, eine Dialysatpumpe (27), die in dem Dialysatrückgewinnungskanal (25) bereitgestellt ist, einen Blutkreislauf (3), der mit dem Dialysegerät (2) verbunden ist, einen Fluidersatzkanal (6), der mit dem Dialysatzufuhrkanal (24) und dem Blutkreislauf (3) verbunden ist, wobei die Fluidersatzpumpe (19) in dem Fluidersatzkanal (6) bereitgestellt ist, und Steuermittel (C) zum Steuern der Dialysatpumpe (27) und der Fluidersatzpumpe (19),
**dadurch gekennzeichnet, dass** das Steuermittel (C) Folgendes ausführt:
einen ersten Vorgang des Betreibens der Dialysatpumpe (27) bei einer vorbestimmten eingestellten Durchflussrate, um eine Gesamt-Ausströmungszeit zu erhalten, bis Dialysat, das zugeführt wird, um die Zufuhrkammer zu füllen, verbraucht ist,
einen zweiten Vorgang des Betreibens der Fluidersatzpumpe (19) bei einer vorbestimmten eingestellten Durchflussrate nur für eine vorbestimmte Zeitdauer, um einen Teil des Dialysats, das zugeführt wurde, um die Zufuhrkammer (21a, 22b) zu füllen, ausströmen zu lassen,
einen dritten Vorgang des Betreibens der Dialysatpumpe (27) bei derselben eingestellten Durchflussrate wie im ersten Vorgang, um die verbleibende Ausströmungszeit zu erhalten, bis restliches Dialysat, das noch in der Zufuhrkammer (21a, 22a) verbleibt, nach Beenden des zweiten Vorgangs verbraucht ist und
einen Fluidersatzpumpen-Kalibrierungsvorgang des Berechnens einer wesentlichen Durchflussrate der Fluidersatzpumpe (19) ausgehend von der Gesamt-Ausströmungszeit und der verbleibenden Ausströmungszeit und Einstellen der Einstellung der Fluidersatzpumpe (19), um eine Differenz in einem Fall zu beseitigen, in dem die Differenz zwischen der eingestellten Durchflussrate und der wesentlichen Durchflussrate in der Fluidersatzpumpe (19) vorliegt.

## Revendications

1. Dispositif d'étalonnage d'une pompe de remplacement de fluide dans un système d'hémodialyse (1) comprenant une chambre de dialysat (21, 22) divisée en une chambre d'alimentation (21a, 22a) et une chambre de récupération (21b, 22b) par un diaphragme (D), un passage d'alimentation en dialysat (24) reliant la chambre d'alimentation (21a, 22a) et un dialyseur (2), un passage de récupération de dialysat (25) reliant le dialyseur (2) et la chambre de récupération (21b, 22b), une pompe de dialysat (27) prévue dans le passage de récupération de dialysat (25), un circuit sanguin (3) relié au dialyseur (2), un passage de remplacement de fluide (6) relié au passage d'alimentation en dialysat (24) et au circuit sanguin (3), une pompe de remplacement de fluide (19) prévue dans le passage de remplacement de fluide (6), et des moyens de commande (C) pour commander la pompe de dialysat (27) et la pompe de remplacement de fluide (19), **caractérisé en ce que** les moyens de commande (C) exécute :
une première opération consistant à faire fonctionner la pompe de dialysat (27) à un débit de consigne prédéterminé pour obtenir un temps de décharge total jusqu'à épuisement du dialysat fourni pour remplir la chambre d'alimentation,
une deuxième opération consistant à faire fonctionner la pompe de remplacement de fluide (19) à un débit de consigne prédéterminé uniquement pendant un temps prédéterminé, pour décharger une partie du dialysat fourni pour remplir la chambre d'alimentation (21a, 22a),
une troisième opération consistant à faire fonctionner la pompe de dialysat (27) au même débit de consigne que lors de la première opération, pour obtenir un temps de décharge restant jusqu'à épuisement du dialysat résiduel restant dans la chambre d'alimentation (21a, 22a) après la fin de la deuxième opération, et
une opération d'étalonnage de pompe de remplacement de fluide consistant à calculer un débit substantiel de la pompe de remplacement de fluide (19) à partir du temps de décharge total et du temps de décharge restant, et ajuster un paramétrage de la pompe de remplacement de fluide (19) pour éliminer une différence dans un cas où il existe la différence entre le débit de consigne et le débit substantiel dans la pompe de remplacement de fluide (19).
